# EUROPEAN PATENT SPECIFICATION

(11) **EP 0 829 244 B1**
(45) Date of publication and mention of the grant of the patent: **26.11.2003**
(21) Application number: 97500150.4
(22) Date of filing: 01.09.1997
(51) Int. Cl.: A61F 2/34

(54) **Reconstruction acetabulum prosthesis**
Acetabulum-Prothese zur Rekonstruktion
Prothèse acétabulaire de reconstruction

(30) Priority: 11.09.1996 ES 9601931
(43) Date of publication of application: 18.03.1998
(73) Proprietor: INDUSTRIAS QUIRURGICAS DE LEVANTE, 46988 Paterna - Valencia (ES)
(72) Inventor: Moleon Camacho, Miguel Angel, Fuente del Jarro 46988 Paterna Valencia (ES); Aguilar Cortes, Francisco, Fuente del Jarro 46988 Paterna Valencia (ES); De Gracia De Castillo De Olivares, Javier, Fuente del Jarro 46988 Paterna Valencia (ES)
(74) Representative: Koepe, Gerd L., Dipl.-Chem.

(56) References cited:
- EP-A- 0 402 810
- EP-A- 0 501 207
- EP-A- 0 605 368
- DE-U- 9 212 420
- US-A- 4 904 265
- US-A- 5 571 198

## Description

This invention refers to a new reconstruction acetabulum prosthesis to be preferably used in hip revision surgery, although its use is also indicated for those cases of primary surgery in which a conventional implant without cementing does not remain stable for any reason.

When a hip prosthesis moves, frequently severe osseous losses appear which settle at the acetabulum level -the term "acetabulum" will be applied to the articular cavity of the pelvis - of the proximal femur or in both structures.

The acetabulum, place for application of the invention, may suffer small osseous losses, not changing in either the solidity nor the morphology thereof, but if this occurs, it does so at an insignificant level; these cases may be solved by means of conventional surgical techniques. However,.major osseous losses of the columns or acetabulum ceiling require the massive contribution of osseous grafts, the morphological changes and the bad osseous quality of the receiving bone being the basic reasons endangering the installation of the implant: the use of threaded cotyles is unsure and the models solely screwed on the base are unstable on many occasions, since the screws cannot be tightened in these acetabula with very deteriorated base and ceiling, or in the grafted areas interposed between the cotyle and the pelvis.

In replacement surgery it is very important that, independently from the technique or type of prosthesis, the fixing of the grafts contributed and the implant is solid; it is the only way to manage that the grafts are colonized by the surrounding live bone, permanently and biologically fixing the newly incorporated implant.

When the primary stability of the implant is not correct, important post-operation problems may appear (cotyle detachment, moving of the grafts, dislocation of the prosthesis, extended time in bed, etc.) or a lack of osteo-integration thereof, producing a fibrous interface between the cotyle and the receiving bone.

Impacted models are not recommendable in these situations, since their design and anchoring method were not created for this purpose. The percussion needed for their installation may break the base of the acetabulum or the very weak osseous columns.

Cemented cotyles, together with mixed techniques of cement grafting, are a desperate solution in many cases, and only recommended in very special occasions.

Cotyles screwed in their base enjoy our confidence, but when osseous loss is very important, it may occur that the screws can not be tightened sufficiently.

To improve primary fixing, a cotylar implant was designed consisting of a metallic ring with some fixed extensions, which is screwed to the iliac, and over said ring, also with screws, the cotyloid component of the implant is fixed. In spite of its advantages, this system is not very versatile and occupies a large space. Moreover, it does not permit an easy reconstruction of the osseous defects, prior to the installation of the ring and very complicated, it being just in this area, changed in structure and form, where osseous reconstruction is necessary.

In turn, a solid fixing of the ring requires a close contact between it and the receiving bone, which in some cases conditions an incorrect position of the ring and, finally, of the cotyle to be fixed on it. Last, the extensions of the ring have a fixed arrangement and size, facts which hinder the positioning of the former and the anchoring on healthy bone.

The document DE 9212420 U describes a new more efficient prosthesis in which the cotyloid metallic rim has been divided into some segments with different arch length or width. Said segments, named flange, can be housed in the corresponding slot performed in the cotyle. Said flanges can be connected to the cotyle by passing screws. On the other hand the flanges, placed radial, extend forming several branches (from 1 to 3) and are provided with screw holes for bone fixation.

The document EP-A 0 605 368 relates to a prosthesis cotyl cup element for reconstructing a hip acetabulum in a hip full recovery operation, for the head portion of a prosthesis stem, comprising a cup element, including a substantially hemispheric recess, delimited by a circular flange. On the flange there are provided a plurality of recesses for engaging therein radially extending elements which can be affixed, by screws, to sound regions of the pelvis of a patient. In an acetabular cup according to document EP-A 0 501 207, for example for a hip joint or a shoulder joint, a basic body comprises a rim extending around its cavity and reception holes in which a component can be fixed. It is to be possible for at least one further component to be fixed in selectable positions on the basic body even after implantation of the latter. For this purpose, the reception holes are made in the rim of the basic body and extend from their orifice into the basic body. These orifices rest against the front face of the rim. A large number of identical reception holes is distributed over 360 ° in the rim.

The present invention relates to a reconstruction acetabulum prosthesis according to claim 1.

The implant designed for this invention basically improves the fixing of the prosthesis cotyle in replacement surgery and facilitates osseous reconstruction. Its anchoring to the healthy periacetabulum bone is possible at many points, at a distance from the osseous defects, by using changeable fixing lips of various shapes and sizes, by which a solid primary fixing is obtained. Once the implant has been installed, chips of a spongy material may be introduced beneath it in the remaining interstices, achieving an excellent repair of the defects. The anchoring of the massive grafts may also be improved by means of screws fixing the bottom of the dome or the lips. These lips may be fixed in any position along the circumference of the rim of the cotylar implant, and it is possible to fix the lips by screwing to the healthy areas of the iliac, hence obtaining a very solid installation, superior to traditional designs.

The design of this invention will be understood better with the aid of the following:

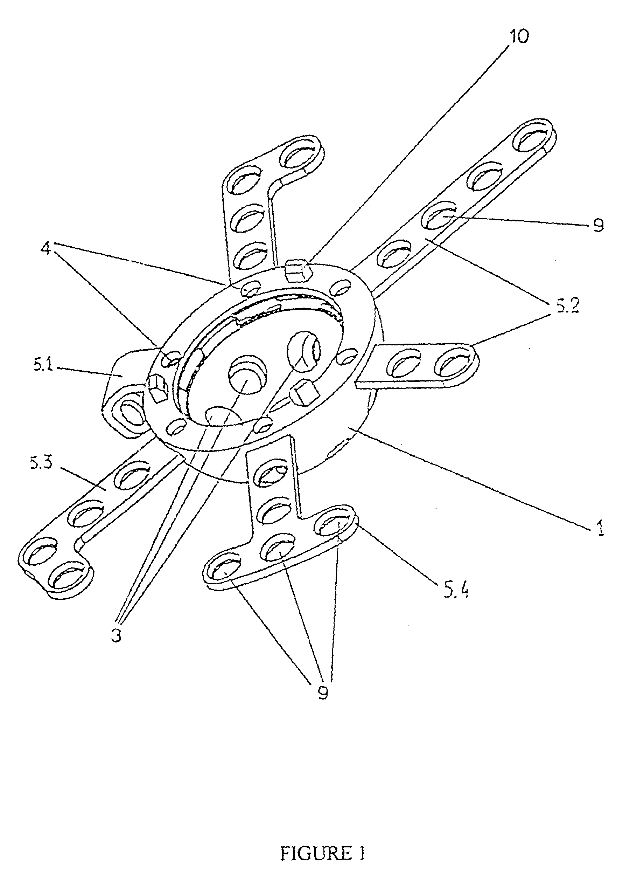

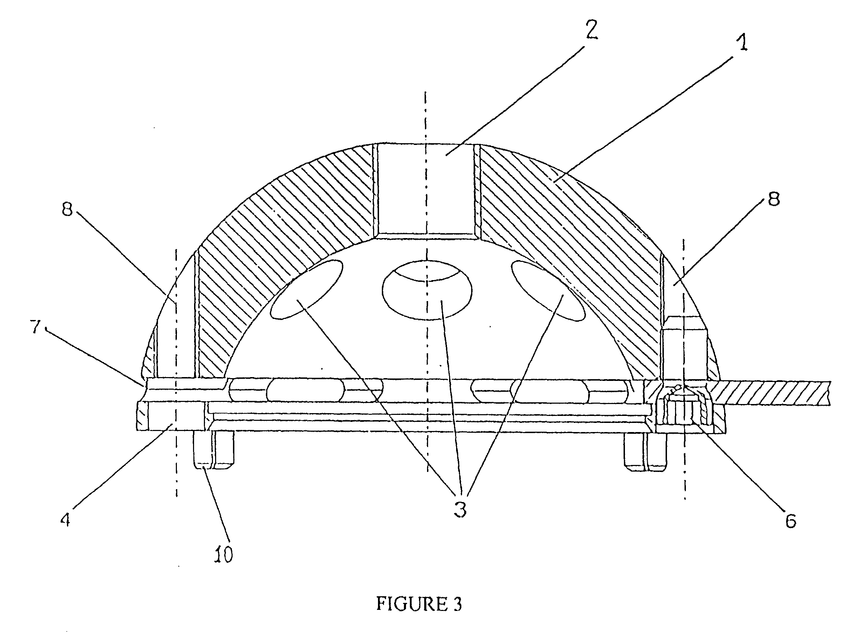

Figure 1.- Lower perspective view of a practical embodiment of the cotyle with a series of fixing lips installed.

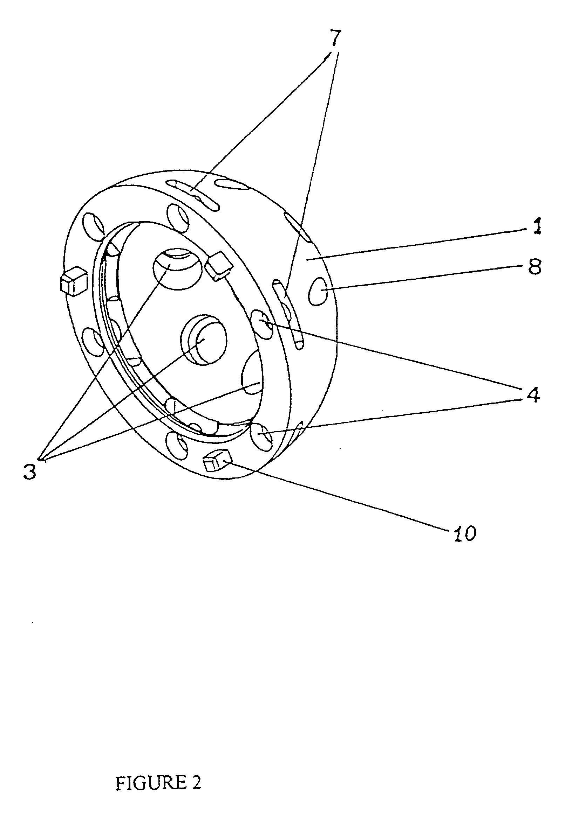

Figure 2.- Lower perspective view of the cotyle, object of this invention.

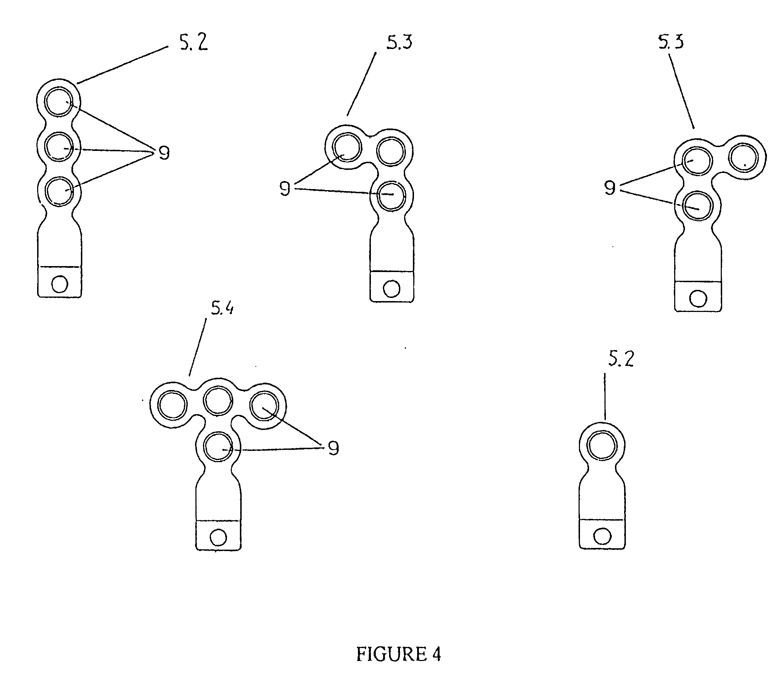

Figure 3.- Section of the cotyle with a detail of the threaded transfixing hole.
Figure 4.- Diagram of some lips for fixing to the cotyle, with some of the possible arrangements of these lips.

The cotyloid implant designed for this invention consists of a hemispherical metallic dome 1 in whose bottom there is a series of holes 3, preferably six, where screws of a spongy anchoring may be placed, and on the periphery grooves 7, arranged to receive the same number of fixing lips 5. These lips 5 are malleable plates, of varying size and morphology which interlock to the iliac by means of spongy screws to the dome with a small screw 6.

The material employed for dome construction, screws 6 and lips 5 will be Ti6A14V preferably. According to the figures, the cotyloid dome 1 has a hemispherical or similar shape. Once fixed, it receives in its interior a very-high molecular weight polyethylene insert of the corresponding size which is easily exchangeable (not shown in the figure).

Observing the dome from its concavity, in its centre one can preferably see a threaded hole 2 or any means of fixing a positioner-impacter. Around the same hole, a series of holes 3 is arranged, preferably six, in the bottom of the implant, destined to receive fixing screws. Taking as a reference a clock face, the location of these holes 3 corresponds to the odd hours, that is 1, 3, 5, 7, 9 and 11 for an amount of six holes.

The housing 3 of the screws is cylindrical in their first position and then countersunk. The latter permits that on tightening the screw, its head uniformly adapts to the dome, there not being an area of tensional overload. The fixing of the screw is very solid. At the same time, once the fixing screw has been installed, the rest of the hole 3 (its first section, the cylindrical part) is sealed by a polyethylene stopper having a double mission: on one hand, if the screw is loosened, it prevents that the rubbing with the polyethylene dome detaches detritus causing granulomas to foreign bodies and being the cause for the implant moving; on the other hand, at the housing level for the screws, areas without support for the polyethylene dome do not remain in the bottom of the cotyle, hence avoiding areas of tensional overload.

The dome rim has, according to the figure and preferably, six small transfixing holes 4, which emerge from the rim along the convexity of the dome 1, crossing the lip housing 5 to the metallic dome 1. Once the transfixing screw 6 is tightened, its head does not protrude on the exchangeable polyethylene dome rim.

Preferably three raised patterns, having the shape of small turrets or similar 10, which are adjusted on the peripheral marks of the polyethylene dome, facilitating a positioning and opposing the rotation thereof.

The convexity of the cotyloid dome (1) has next to the rim preferably six rectangular grooves (7), arranged alternating in relation to the bottom holes (3), being located where on a clock face the even hours are, that is, 2, 4, 6, 8, 10 and 12.

These grooves 7 house the fixing lips 5 and the small transfixing screw 6 which interlocks the dome 1 with the lips 5. Behind each groove 7 there is a small hole corresponding to the exit of the threaded channel 8 housing said transfixing screw.

In the vertex of the dome convexity 1, there is a centre hole 2 to house the positioner-impacter. This dome 1 has a porous coating of pure titanium sintered to the substrate in a vacuum oven. This porous coat of titanium facilitates the colonization of the implant by the surrounding bone, guaranteeing a long term biological fixing.

The fixing lips 5 will preferably be made of Ti6A14V, of different shapes and lengths, acting as joining ties between the metallic dome 1 and the bone surrounding it. Due to its design versatility and the different positions in which it may be placed, they anchor on healthy areas of iliac bone, where the spongy screws fixing them grip well.

According to the characteristics of the case, two or more fixing lips 5 may be used until a firm fixing of the prosthesic cotyle is obtained.

These lips 5 have a small hole for the passage of the transfixing screw 6 fixing the plate or lip to the metallic dome (1) at their rectangular or similarly shaped end. This end will preferably have an elbow at 80° to facilitate the adaptation of the fixing lip 5 to the iliac blade; and to the acetabulum columns.

The holes 9 to house the spongy screws fixing to the bone are countersunk, with the purpose of facilitating the adaptation of their heads to the plate. Their thickness will be sufficient (approximately 2 mm) to permit easy adapting to the receiving bone having a complicated morphology.

The lips 5 shape a narrowing between the screw location holes 9, so that they facilitate changes of shape on shaping them, said shaping occurring at this level and not in the holes 9, which would cause the appearance of breakages in this area.

The shape and size of the lips 5 may be varied for the purposes to be met; among the most significant shapes, the following may be mentioned:
- Hook shaped lip (5.1) , for anchoring on the stopper frame or on the acetabulum columns.
- Short and long straight lips (5.2), for anchoring on the columns or on the iliac plate.
- Right and left "L" shaped lips (5.3) destined to the iliac blade.
- "T" shaped lip (5.4), similar to the "L" shaped lip.

Any model of lip 5 may be fitted in each groove 7 of the dome 1, which, due to the harmonic distribution of the anchoring holes 3 to the bottom and housing grooves 7, does not have to be situated in a determined position, but in the most convenient to solve the case. These qualities endow the system with an enormous versatility and ease of fitting.

The joint between the fixing plates 5 and the dome 1 is double : on one hand, the rectangular groove 7 of the dome, receives the lip 5 opposing movements in the transverse, front and rotation planes. On the other hand, the small fixing screw 6 acts like as a bolt, threaded in the dome 1 and transfixing the lip 5, basically opposing ascending and descending movements between both system components.

Last, the friction surface is a dome 1 made of very high molecular weight polyethylene, fitted by simple pressure, covering the metallic component, once in place.

There is an asymmetric and another symmetric component, with anti-luxation lip. The peripheral fittings facilitate their positioning and oppose the rotations between both domes, as they remain anchored in the metallic turrets 10 of the cotyle rim.

Therefor, a prosthesic cotyle is presented consisting of two components:
1.- Hemispheric metallic dome 1 with a porous coating.
2.- Exchangeable peripheral fixing lips 5.

The dome 1 has preferably six holes 3 of a special design permitting their fixing by means of screws. Over the heads of the latter, a polyethylene stopper is fixed to seal the holes 3.

The lips 5 are malleable plates of different shapes and sizes, that may be fitted according to the requirements in each one of the grooves 7 in the dome 1.

The assembly of both components is executed with a transfixing screw 6 which threads in the dome.

The versatility of the system and the possibility of achieving several anchoring points by means of its movable lips reaching the healthy periacetabulum bone obtains a very solid primary fixing of the implant, facilitating osseous reconstruction, improving the post-operation phase and favoring the long term fixing of the implant.

Once the nature of this invention and its practical application have been sufficiently described, it only remains to be said that both its form and materials and the execution thereof are susceptible of modifications, provided that they do not affect the characteristics claimed below.

## Claims

1. Reconstruction acetabulum prosthesis, consisting of
- a hemispheral dome (1), said dome (1) being provided
- with holes (3) in its bottom suitable for receiving bottom spongy screws;
- with transfixing holes (4) at the dome rim;
- with corresponding threaded channels (8) at the convexity of the dome suitable for receiving transfixing screws (6);
- next to said dome rim, with peripheral grooves (7) along the circumference of the rim suitable for housing changeable fixing lip(s) (5); and
one or more than one changeable fixing lip(s) (5) having the form of plates of different shapes and sizes and capable of fixing said reconstruction acetabulum prosthesis to the periacetabulum iliac bone **characterized in that** each fixing lip is adapted to be fitted into each peripheral groove (7) and to be locked therein by means of transfixing screws (6);
- said hemispheral dome (1) being provided with an external porous coating; and
- all peripheral grooves (7) having identical shape and size.

2. The reconstruction acetabulum prosthesis of claim 1, wherein the peripheral grooves (7) are rectangular grooves.

3. The reconstruction acetabulum prosthesis of claim 1 or claim 2, wherein said holes (3) in the bottom of said hemispheral dome (1) are cylindrical in their first section and countersunk in its bottom so as to be suitable to receive spongy screws and their sealing, by means of a polyethylene stopper, once the screws have been fixed.

4. The reconstruction acetabulum prosthesis of any of claims 1 to 3, wherein the distribution of said holes (3) in the bottom of said hemispheral dome (1) is harmonic with respect to the bottom, preferably and taking as a reference a clock face for an amount of six holes, the position of six holes (3) corresponds to the odd hours.

5. The reconstruction acetabulum prosthesis of any of claims 1 to 4, wherein the position of said identical shape and size rectangular peripheral grooves (7) around said dome rim is alternating in relation to the bottom holes (3), preferably and taking as a reference a clock face for an amount of six grooves, the position of six grooves (7) corresponds to the even hours.

6. The reconstruction acetabulum prosthesis of any of claims 1 to 5, wherein said changeable fixing lip(s) (5) is/are malleable to be perfectly adapted to the anatomy of the intervention place and the requirements of surgical operations and has/have shapes of a hook (5.1), of a short or long straight line (5.2), of a right-angle or left-angle "L" (5.3) or of a "T" (5.4).

7. The reconstruction acetabulum prosthesis of any of claims 1 to 6, wherein said changeable fixing lip(s) (5) has/have a hole (4) at its end fitted into one of said identical shape and size rectangular peripheral grooves (7) and locked therein by means of (a) transfixing screw(s) (6) and allowing to assemble and lock said fixing lip(s) (5) to said hemispheral dome (1).

8. The reconstruction acetabulum prosthesis of any of claims 1 to 7, wherein said changeable fixing lip(s) (5) has/have one or more than one hole(s) (9) for housing spongy screws allowing a fixing of said changeable fixing lip(s) (5) to the periacetabulum iliac bone.

9. The reconstruction acetabulum prosthesis of any of claims 1 to 8, wherein said changeable fixing lip(s) (5) comprise one or several narrowings between the spongy screw location holes (9) so as to facilitate a shaping adaptation of said fixing lip(s) (5) to a complicated morphology of the bone to which said lip(s) (5) is/are to be fixed.

## Patentansprüche

1. Acetabulum-Prothese zur Rekonstruktion, bestehend aus
- einer hemisphärischen Haube (1), wobei die Haube (1) versehen ist
- in ihrer Unterseite mit Löchern (3), die zur Aufnahme von Unterseiten-Schwamm-Schrauben geeignet sind;
- mit Befestigungsdurchgangs-Löchern (4) am Haubenrand;
- mit entsprechenden, mit einem Gewinde versehenen Kanälen (8) auf der konvexen Wölbung der Haube, die zur Aufnahme von Befestigungsdurchgangs-Schrauben (6) geeignet sind;
- mit peripheren Rillen (7) entlang dem Außenumfang des Randes in der Nähe des Haubenrandes, die geeignet sind zur Unterbringung (eines) austauschbaren/r Fixierstreifen(s) (5);
- wobei ein oder mehr als ein austauschbarer Fixierstreifen (5) die Form von Platten unterschiedlicher Formen und Größen aufweist und in der Lage ist, die Acetabulum-Prothese zur Rekonstruktion an dem Periacetabulum-Hüft-Knochen zu fixieren;
- **dadurch gekennzeichnet, daß** jeder Fixierstreifen daran angepaßt ist, in jede periphere Rille (7) eingepaßt zu werden und darin mittels der Befestigungsdurchgangs-Schrauben (6) befestigt zu werden;
- wobei die hemisphärische Haube (1) mit einer porösen Außenbeschichtung versehen ist und
- alle peripheren Rillen (7) identische Form und Größe haben.

2. Acetabulum-Prothese zur Rekonstruktion nach Anspruch 1, worin die peripheren Rillen (7) rechteckige Rillen sind.

3. Acetabulum-Prothese zur Rekonstruktion nach Anspruch 1 oder Anspruch 2, worin die Löcher (3) in der Unterseite der hemisphärischen Haube (1) in ihrem ersten Abschnitt zylindrisch sind und in ihrem unteren Bereich versenkt sind, so daß sie geeignet sind, Schwammschrauben und ihren Verschluß mittels eines Polyethylen-Stopfens aufzunehmen, sobald die Schrauben fixiert wurden.

4. Acetabulum-Prothese zur Rekonstruktion nach einem der Ansprüche 1 bis 3, worin die Verteilung der Löcher (3) in der Unterseite der hemisphärischen Haube (1) harmonisch in Bezug auf die Unterseite ist, wobei vorzugsweise und unter Bezugnahme auf ein Zifferblatt für eine Zahl von sechs Löchern die Position von sechs Löchern (3) den ungeraden Stunden entspricht.

5. Acetabulum-Prothese zur Rekonstruktion nach einem der Ansprüche 1 bis 4, worin die Position der identische Form und Größe aufweisenden rechteckigen peripheren Rillen (7) um den Rand der Haube herum in Bezug auf die Unterseiten-Löcher (3) alterniert, wobei vorzugsweise und unter Bezugnahme auf ein Zifferblatt für eine Zahl von sechs Rillen die Position von sechs Rillen (7) den geraden Stunden entspricht.

6. Acetabulum-Prothese zur Rekonstruktion nach einem der Ansprüche 1 bis 5, worin der/die austauschbare(n) Fixierstreifen (5) biegbar ist/sind, um perfekt an die Anatomie des Eingriffsortes und die Erfordernisse von chirurgischen Operationen angepaßt zu werden, und die Form eines Hakens (5.1), einer kurzen oder langen geraden Linie (5.2), eines einen Winkel nach rechts oder einen Winkel nach links aufweisenden "L" (5.3) oder eines "T" (5.4) aufweist/aufweisen.

7. Acetabulum-Prothese zur Rekonstruktion nach einem der Ansprüche 1 bis 6, worin der/die austauschbare(n) Fixierstreifen (5) ein Loch (4) an seinem/ihrem Ende aufweist/aufweisen, das in eine der identische Form und Größe aufweisenden rechtekkigen peripheren Rillen (7) eingepaßt und darin mittels (einer) Befestigungsdurchgangs-Schraube(n) (7) befestigt wird, und worin dies ermöglicht, daß der/die Fixierstreifen (5) an der hemisphärischen Haube (1) angebracht und befestigt wird/werden.

8. Acetabulum-Prothese zur Rekonstruktion nach einem der Ansprüche 1 bis 7, worin der/die austauschbare(n) Fixierstreifen (5) ein oder mehr als ein Loch/Löcher (9) zum Unterbringen von Schwamm-Schrauben aufweist/aufweisen, die ein Fixieren des/der austauschbaren Fixierstreifen(s) (5) an dem Periacetabulum-Hüftknochen erlauben.

9. Acetabulum-Prothese zur Rekonstruktion nach einem der Ansprüche 1 bis 8, worin der/die austauschbare(n) Fixierstreifen (5) eine oder einige Einschnürung(en) zwischen den Löchern (9) für die Anordnung der Schwamm-Schrauben umfassen, um ein formendes Anpassen des/der Fixierstreifen(s) (5) an eine komplizierte Morphologie des Knochens zu erleichtern, an dem der/die Streifen (5) befestigt werden soll(en).

## Revendications

1. Prothèse de reconstruction de l'acétabulum consistant en :
- une coupole hémisphérique (1), ladite coupole (1) étant pourvu
- de trous (3) dans son fond adaptés pour recevoir des vis spongicuses de fond ;
- avec des trous transfixants (4) sur le bord de la coupole ;
- avec des canaux (8) filetés de manière correspondante dans la partie convexe de la coupole adaptés pour recevoir les vis transfixantes (6) ;
- à côté dudit bord de la coupole, des rainures périphériques (7) le long de la circonférence de la coupole adaptées pour loger un/des rebord(s) de fixation (5) interchangeable(s) et ;
- un ou plus d'un rebord(s) de fixation interchangeable(s) (5) ayant la forme de plateaux de différentes formes et différentes tailles et étant capable de fixer ladite prothèse de reconstruction de l'acétabulum à l'os iliac du périacétabulum
- **caractérisé en ce que** chaque rebord de fixation est adapté pour être ajuster dans chaque rainure périphérique (7) et pour être verrouillé à l'intérieur de cette rainure aux moyens de vis transfixantes (6) ;
- ladite coupole hémisphérique (1) étant pourvue d'un revêtement externe poreux ; et
toutes les rainures périphériques (7) ayant une forme et une taille identique.

2. La prothèse de reconstruction de l'acétabulum selon la revendication 1, dans laquelle les rainures périphériques (7) sont des rainures rectangulaires.

3. La prothèse de reconstruction de l'acétabulum selon la revendication 1 ou la revendication 2, dans laquelle lesdits trous (3) dans le fond de ladite coupole hémisphérique (1) sont cylindriques dans leur première section et fraisés dans leur partie inférieure afin d'être adaptés à recevoir des vis spongieuses et leurs garnitures étanches, aux moyens de bouchons de polyéthylène, une fois que les vis ont été fixées.

4. La prothèse de reconstruction de l'acétabulum selon l'une quelconque des revendications 1 à 3, dans laquelle la distribution desdits trous (3) dans le fond de ladite coupole hémisphérique (1) est harmonique par rapport au fond, de préférence et prenant comme référence la face d'une montre pour une quantité de six trous, la position des six trous (3) correspond aux heures impaires.

5. La prothèse de reconstruction de l'acétabulum selon l'une quelconque des revendications 1 à 4, dans laquelle la position desdites rainures (7) périphériques rectangulaires de taille et de forme identique autour dudit bord de la coupole alterne par rapport aux trous du fond (3), de préférence et prenant comme référence la face d'une montre pour une quantité de six rainures, la position des six rainures (7) correspondent aux heures paires.

6. La prothèse de reconstruction de l'acétabulum selon l'une quelconque des revendications 1 à 5, dans laquelle le/lesdits rebord(s) de fixation interchangeable(s) est/sont malléables pour être parfaitement adaptés à l'anatomie de la place de l'intervention et aux exigences des opérations chirurgicales et a/ont des formes de crochet (5.1), de ligne droite, courte ou longue (5.2), d'un angle à droit ou d'un angle à gauche en forme de "L" (5.3) ou une forme de "T" (5.4).

7. La prothèse de reconstruction de l'acétabulum selon l'une quelconque des revendications 1 à 6, dans laquelle le/lesdits rebord(s) de fixation interchangeable(s) (5) a/ont un trou (4) à leur extrémité ajusté dans l'une desdites rainures (7) périphériques rectangulaires de taille et de forme identique et verrouillé à l'intérieur aux moyens de vis transfixantes (6) et permettant d'assembler et de verrouiller le/lesdits rebord(s) de fixation (5) à ladite coupole hémisphérique (1).

8. La prothèse de reconstruction de l'acétabulum selon l'une quelconque des revendications 1 à 7, dans laquelle le/lesdits rebord(s) de fixation interchangeable(s) (5) a/ont un ou plus d'un trou(s) (9) pour loger des vis spongieuses permettant la fixation du/desdit(s) rebords de fixation interchangeable(s) à l'os iliac du péricetabulum.

9. La prothèse de reconstruction de l'acétabulum selon l'une quelconque des revendications 1 à 8, dans laquelle le/lesdits rebord(s) de fixation interchangeable(s) (5) comprennent un ou plusieurs rétrécissements entre les trous de logement des vis spongieuses (9) afin de faciliter l'adaptation de la forme du/desdits rebord(s) de fixation (5) à une morphologie complexe de l'os à laquelle le/lesdits rebord(s) (5) doivent être fixés.
